# EUROPÄISCHE PATENTSCHRIFT

(11) **EP 1 827 319 B1**
(45) Veröffentlichungstag und Bekanntmachung des Hinweises auf die Patenterteilung: **26.08.2009**
(21) Anmeldenummer: 05821403.2
(22) Anmeldetag: 30.11.2005
(51) Int. Cl.: A61F 2/42, A61F 2/58

(54) **KÜNSTLICHES GELENKELEMENT SOWIE EIN DAMIT AUSGESTATTETES GREIFWERKZEUG**
ARTIFICIAL JOINT ELEMENT AND GRIPPING TOOL EQUIPPED WITH THE SAME
ELEMENT D'ARTICULATION ARTIFICIEL ET OUTIL DE PREHENSION DOTE DE CET ELEMENT

(30) Priorität: 03.12.2004 DE 102004058546; 02.05.2005 DE 102005020779
(43) Veröffentlichungstag der Anmeldung: 05.09.2007
(73) Patentinhaber: Aequos Endoprothetik GmbH, 81925 München (DE)
(72) Erfinder: MEESENBURG, Larissa, 37073 Göttingen (DE); NÄGERL, Hans, 37130 Gleichen (DE)
(74) Vertreter: Scheffler, Jörg
(86) Internationale Anmeldenummer: PCT/DE2005/002162
(87) Internationale Veröffentlichungsnummer: WO 2006/060992

(56) Entgegenhaltungen:
- WO-A-92/00709
- DE-A1- 3 045 777
- DE-A1- 10 237 373
- FR-A- 2 742 043
- FR-A- 2 743 717
- US-A1- 2002 065 561
- BEEVERS D J ET AL: "DESIGN OF A NON-CONSTRAINED, NON-CEMENTED, MODULAR, METACARPOPHALANGEAL PROSTHESIS" PROCEEDINGS OF THE INSTITUTION OF MECHANICAL ENGINEERS. JOURNAL OF ENGINEERING IN MEDICINE. PART H, MECHANICAL ENGINEERING PUBLICATIONS LTD, LONDON, GB, Bd. 209, Nr. H3, 22. Januar 1996 (1996-01-22), Seiten 185-195, XP000582788 ISSN: 0954-4119

## Beschreibung

Die Erfindung betrifft ein künstliches Gelenkelement zum Einsatz bei einem menschlichen Fingergelenk, mit einer im Wesentlichen konkaven Gelenkschale und mit einem im Wesentlichen konvexen Gelenkkopf. Weiterhin betrifft die Erfindung ein mit einem solchen Gelenkelement ausgestattetes Greifwerkzeug.

Ein solches künstliches Gelenkelement ist beispielsweise durch die US 56 74 297 A bekannt, die ein künstliches Fingergelenk mit einem konvexen Gelenkkopf und mit einer konkaven Gelenkschale beschreibt, die unabhängig voneinander jeweils mit einem Schaft in einem Knochenende befestigbar sind und in einer Artikulationsebene aus einer Extensionsstellung mit parallelen Schaftachsen in eine Hyperextensionsstellung oder in eine Artikulationsendstellung beweglich sind. Der Gelenkkopf und die Gelenkschale bilden dabei ein Schamiergelenk.

Auch die FR 2 743 717 A1 beschreibt bereits ein künstliches Gelenkelement zum Einsatz eines oder mehrerer Einzelgelenke in einem aus einer Kombination von mehreren kinematisch gekoppelten Einzelgelenken bestehenden Gelenksystem, insbesondere zum Einsatz bei einem menschlichen Fingergelenk, wobei jedes Gelenkelement mit einer im Wesentlichen konkaven Gelenkschale und mit einem im Wesentlichen konvexen Gelenkkopf ausgestattet ist. Das Gelenkelement weist zumindest vier Freiheitsgrade auf und hat zum Ersatz des Proximalen Interphalangeal-Gelenkes (PIP) und/oder des Distalen Interphalangeal-Gelenkes (DIP) einen Gelenkkopf mit zwei durch einen konkaven Bereich verbundenen konvexen Bereichen.

Die DE 102 37 373 A1 betrifft ein künstliches Gelenk, insbesondere einer künstlichen Hand, bestehend aus mehreren, gelenkig miteinander verbundenen Gliedern. Die einzelnen Glieder sind mittels eines elastischen Verbindungselementes um mehrere Achsen schwenkbar miteinander verbunden, sodass jeweils eine mittels eines Zugmittels eingeleitete Zugkraft zum Beugen und zum Strecken des jeweiligen Gliedes vorgesehen ist. Mittels dieses Zugmittels kann der Gelenkspalt zwischen den benachbarten Gelenkflächen bedarfsweise derart verringert werden, dass die jeweils gewünschte Anzahl von Freiheitsgraden eingestellt und zugleich eine Versteifung des Gelenkes erreicht wird.

Weiterhin zeigt die US 42 31 121 ein Fingergelenk, das aus einem als Kugelkopf ausgeführten Gelenkkopf mit angefügtem Schaft und aus einer kugelschalenförmigen Gelenkschale mit ebenfalls angeformten Schaft besteht. Der Gelenkkopf und die Gelenkschale weisen dadurch eine gemeinsame Artikulationsfläche auf.

Ein künstliches Gelenkelement ist auch Gegenstand der EP 11 360 47 A1, die ein künstliches Fingergelenk mit einem konvexen Gelenkkopf und mit einer konkaven Gelenkschale beschreibt. Der Gelenkkopf und die Gelenkschale sind unabhängig voneinander jeweils mittels eines Schaftes in einem Knochenende befestigbar und aus einer Extensionsstellung mit parallelen Schaftachsen in eine Hyperextensionsstellung oder in eine Artikulationsendstellung bewegbar. Durch Anbringen eines zweiten Anschlages in einer Stellung, in der ein erster Anschlag für die Hyperextensionsstellung erreicht wird und ein Momentanzentrum für eine Fortsetzung der Drehung bilden würde, wird die Bewegung gestoppt und dadurch ein Abgleiten des Gelenkkopfes von der Gelenkschale vermieden.

Aus der DE 43 37 922 A1 ist eine Fingergelenkprothese bekannt, von deren Gelenkschale ein Zapfen absteht, der an seinem freien Ende eine Kugel trägt, die mit der Gelenkschale eine Artikulationsfläche ausbildet. Die Kugel wird dabei verriegelnd hintergriffen, um ein unerwünschtes seitliches Abgleiten zu vermeiden.

Die EP 07 36 293 A1 beschreibt weiterhin auch ein künstliches Fingergelenk mit einem ersten und einem zweiten hohlen Verankerungsvorsprung, der in die Fingerröhrenknochen einsetzbar ist. Zwischen den Verankerungsvorsprüngen ist ein Scharniergelenk in Form eines Kugelgelenkes angeordnet, wobei der erste Verankerungsvorsprung mit einem Kugelkäfig des Scharniergelenkes verbunden ist, in weichem eine mit dem anderen Verankerungsvorsprung versehene Gelenkkugel gelagert ist. Der Verankerungsvorsprung tritt durch einen Schlitz des Kugelkäfigs hindurch und steht mit dem zweiten Verankerungsvorsprung in Verbindung, wobei sich der Schlitz im Kugelkäfig in Streckstellung des Gelenkes zu der Stelle an welcher der Verankerungsvorsprung den Kugelkäfig in Beugestellung des Gelenks durchtritt, stetig aufweitet.

Die DE 90 05 372 U1 offenbart auch bereits eine insbesondere für ein Fingergelenk bestimmte Gelenkteilprothese mit einem eine Lagerfläche aufweisenden Lagerkörper und einem in den Knochen einzubettenden Schaft. Der Lagerkörper ist dabei als dünnwandiges Schalenelement aus hochfestem metallischem Werkstoff mit einer Dicke von maximal 1 mm ausgebildet. Die Lagerfläche ist derjenigen eines menschlichen Fingergelenks funktionell in idealisierter Form nachgebildet und lässt ähnlich dem natürlichen Fingergelenk außer der Beugebewegung kleine Lateralbewegungen relativ zu dem anderen Gelenkteil zu.

Ferner betriff auch die DE 196 50 816 A1 ein künstliches Fingergelenk. Um bei einem Strecksehnenabriss den Finger wieder in die gestreckte Lage zu bringen sind die mit jeweils einem Knochen verbundenen Gelenkteile mittels eines flexiblen Verbindungselementes verbunden, welche eine der Beugung entgegenwirkende Rückstellkraft entwickelt

Als nachteilig erweist sich bei allen bisher bekannten künstlichen Gelenkelementen die damit verbundene Bewegungsfähigkeit des Gelenkes, welches die natürlichen Gelenkeigenschaften nur eingeschränkt nachzubilden vermag. Aufgrund der derart abweichenden Gelenkeigenschaften wird das künstliche Gelenk von dem Patienten oftmals bereits aufgrund des damit einhergehenden Bewegungsablaufes als Fremdkörper und daher als störend empfunden.

Außerdem ist durch die DE 102 31 538 C1 ein künstliches Gelenk bekannt, umfassend ein erstes, durch einen ersten Gelenkkopf und eine erste Gelenkpfanne gebildetes Gelenkkompartiment und ein zweites durch einen zweiten Gelenkkopf und eine zweite Gelenkpfanne gebildetes Gelenkkompartiment, wobei die Kontaktflächen der jeweiligen Gelenkkompartimente in der Hauptfunktionsebene einen Versatz aufweisen. Um die Eigenschaften des künstlichen Gelenkes zu verbessern, sind die Kontaktflächen der beiden Gelenkkompartimente derart geneigt angeordnet, dass die Flächennormalen der Kontaktflächen bei jedem Flexionswinkel einen gemeinsamen Schnittpunkt aufweisen.

Die US 2002/0065561 A zeigt ein Gelenkelement gemäß der Präambel von Anspruch 1.

Der Erfindung liegt daher die Aufgabe zugrunde, eine Möglichkeit zu schaffen, ein dem natürlichen Bewegungsablauf wesentlich angenähertes Gelenkelement umfassend eine Gelenkschale und einen Gelenkkopf zu schaffen. Insbesondere sollen dabei die das Bewegungsverhalten wesentlich bestimmenden Freiheitsgrade entsprechend angepasst werden. Weiterhin liegt der Erfindung die Aufgabe zugrunde, ein mit einem solchen künstlichen Gelenkelement ausgestattetes Greifwerkzeug zu schaffen.

Die erstgenannte Aufgabe wird erfindungsgemäß mit einem künstlichen Gelenkelement gemäß den Merkmalen des Anspruchs 1 gelöst. Die Unteransprüche betreffen besonders zweckmäßige Weiterbildungen der Erfindung.

Erfindungsgemäß ist also ein künstliches Gelenkelement vorgesehen, bei dem der Gelenkkopf sowie die Gelenkschale jeweils zwei Kontaktflächen aufweisen, die in dem konvexen Bereich zwischen dem konkaven Bereich und der maximalen distalen Erstreckung liegen. Die Erfindung geht dabei von der Erkenntnis aus, dass die Bewegungseigenschaften des künstlichen Gelenkes dem natürlichen Verhalten dann in optimaler Weise angenähert werden können, wenn das künstliche Gelenk über die mit einem nach dem Stand der Technik bekannten Scharniergelenk realisierbaren Freiheitsgrade zusätzliche Freiheitsgrade und damit eine deutlich abweichende kinematische Kopplung gestattet. Insbesondere die relative Verlagerung der Gelenkteile in der Transversalebene führt dabei zu einer Rückstellkraft, die einer Aufhebung des Kontaktbereichs entgegenwirkt. Der von dem durch die beiden konvexen Bereiche eingeschlossenen Grund abweichende Kontaktlinienverlauf führt daher zu einer Selbststabilisierung des derart ausgestalteten Gelenkelementes.

Dabei hat gemäß einer praktischen Ausgestaltung die konkave Gelenkschale eine auf ihrer Oberfläche verlaufende erste gekrümmte Kontaktlinie mit einem ersten Radius und der konvexe Gelenkkopf eine auf seiner Oberfläche verlaufende zweite gekrümmte Kontaktlinie mit einem zweiten Radius, wobei der erste Radius der ersten gekrümmten Kontaktlinie zumindest in einem Teilbereich zwischen den Extremlagen der Gelenkstellung größer als der zweite Radius der zweiten gekrümmte Kontaktlinie ist.

Hierdurch wird also eine gezielte Inkongruenz des Gelenkkopfs gegenüber der Gelenkschale vorgesehen, durch die sich insbesondere ein linien- oder punktförmiger Kontaktbereich ausbildet. Aufgrund der zusätzlichen Freiheitsgrade wird zugleich bei insbesondere stoßförmigen Belastungsspitzen eine Beschädigung des künstlichen Gelenkes vermieden, weil dabei eine Ausweichbewegung des Gelenkes ermöglicht wird. Zudem ergeben sich aufgrund der zwei derart realisierten Rotationswinkel wesentlich abweichende Kräfteverhältnisse aufgrund der veränderten Hebelarme, sodass bereits geringfügige Änderungen der Bewegungskinematik zu großen Änderungen der Muskelkräfte führen. Insbesondere ist also sowohl eine Rollbewegung um den Kontaktpunkt zwischen den beiden Kontaktlinien als auch eine Gleitbewegung durch eine Drehbewegung um den Mittelpunkt des der Gelenkschale zugeordneten Radius oder des dem Gelenkkopf zugeordneten Radius denkbar. Weiterhin wird im Zusammenhang mit einer den Spalt füllenden Gelenkflüssigkeit aufgrund des Überströmens bzw. der Verdrängung der Flüssigkeit aufgrund der Änderung des Gelenkspaltes in Abhängigkeit der jeweiligen Gelenkbewegung eine zusätzliche Kraftkomponente erzeugt, die beispielsweise zu einer Reduzierung der Flächenpressung und damit zu einem verbesserten Verschleißverhalten führt.

Eine besonders vorteilhafte Ausführungsform der vorliegenden Erfindung wird dadurch erreicht, dass die Differenz zwischen dem ersten Radius und dem zweiten Radius zumindest 1 mm, insbesondere zwischen 2 mm und 4 mm beträgt. Hierdurch wird ein optimales Bewegungsverhalten aufgrund der nach ihrem Betrag durch die Radiendifferenz bestimmten Freiheitsgrade erreicht, wobei die Radiendifferenz des MCP gegenüber dem DIP sowie die Radiendifferenz des PIP gegenüber dem DIP jeweils zunimmt. Dabei sind die Differenzen insbesondere abhängig von der Zuordnung zu den unterschiedlichen Fingergelenken, wobei sich für das Metacarpophalangeal-Gelenk (MCP) eine Radiendifferenz von 3,8 mm, für das proximale Interphalangeal-Gelenk (PIP) eine Radiendifferenz von 1,3 mm sowie für das distale Interphalangeal-Gelenk (DIP) eine Radiendifferenz von 1 mm ergibt.

Besonders praxisgerecht ist auch eine Ausgestaltung der vorliegenden Erfindung, bei der die Differenz zwischen dem ersten Radius und dem zweiten Radius in der gebeugten Gelenkstellung größer als in der gestreckten Gelenkstellung ist, sodass die Relativbewegung der Gelenkteile auch durch die jeweilige Winkelstellung bestimmt ist. Insbesondere ist also die Beweglichkeit aufgrund der zunehmenden Inkongruenz in der gebeugten Stellung erhöht. Hierzu ist der konvexe Gelenkkopf aus zumindest zwei Kugelsegmenten zusammengesetzt, die im Bereich einer gemeinsamen Tangente aufeinander treffend ausgeführt sind und dadurch einen stetigen Bewegungsablauf ermöglichen.

Weiterhin ist es besonders vorteilhaft, wenn die Flächennormalen der Kontaktflächen bei jedem Flexionswinkel einen gemeinsamen Schnittpunkt aufweisen. Durch diese Anordnung der Kontaktflächen wird eine Selbststabilisierung des Gelenkes sowohl bei der Einleitung einer Rotationsbewegung bzw. Torsion als auch bei Einleitung seitlicher Kräfte erreicht.

Die Gelenkschale und der Gelenkkopf könnten jeweils als ein massives Funktionselement mit einem Verbindungsbereich für den Fingerknochen ausgeführt sein. Besonders zweckmäßig ist hingegen eine Abwandlung der vorliegenden Erfindung, bei der das Gelenkelement als ein dünnes Formteil mit einer Materialstärke zwischen 1 mm und 5 mm ausgeführt ist. Hierdurch wird eine wesentliche Reduzierung des Knochensubstanzverlustes erreicht, die es zudem gestattet, funktionsfähige Teilbereiche des natürlichen Gelenkes weitgehend zu erhalten.

Eine andere, ebenfalls Praxis gerechte Gestaltung wird abweichend von dem Einsatz bei menschlichen oder tierischen Gelenken dann erreicht, wenn ein mit zumindest einem künstlichen Gelenk nach einem oder mehreren der vorstehenden Ansprüche ausgestattetes Greifwerkzeug, mit einem durch eine Antriebseinheit verbundene Zugmittel betätigbar ist. Hierdurch wird ein technisches Greifwerkzeug realisiert, welches zugleich eine einfache Steuerung des Bewegungsablaufs als auch eine hohe Reaktionsgeschwindigkeit bei großer Kraftübertragung gestattet.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig.1: ein Gelenksystem mit mehreren kinematisch gekoppelten Einzelgelenken in einem sagittalen Schnitt;
- Fig.2: das in Figur 1 gezeigte Gelenksystem in einem horizontalen Schnitt;
- Fig.3: einen horizontalen Schnitt eines Gelenkelementes des in Figur 2 gezeigten Gelenksystems in einer vergrößerten Darstellung;
- Fig.4: einen entlang der Linie B in Figur 3 geschnittene Ansicht einer konkaven Gelenkschale des Gelenkelementes.
- Fig. 5: einen sagittalen Schnitt eines Gelenkelementes des in Figur 1 gezeigten Gelenksystem in einer vergrößerten Darstellung;
- Fig.6: einen horizontalen Schnitt einer konkaven Gelenkschale des in Figur 5 gezeigten Gelenkelementes;

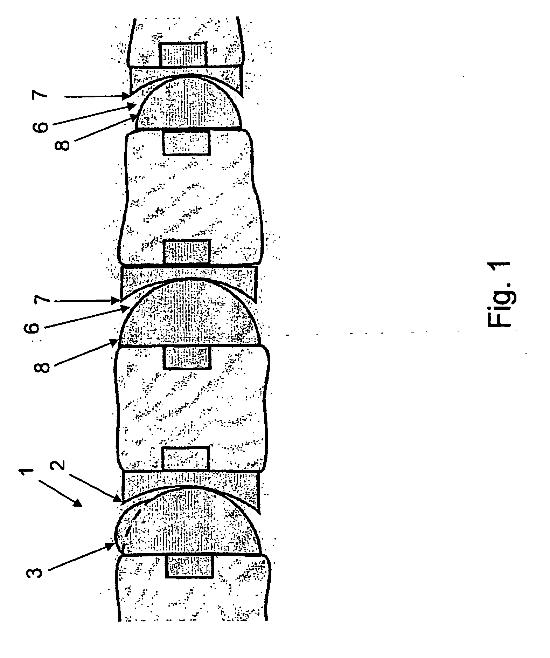

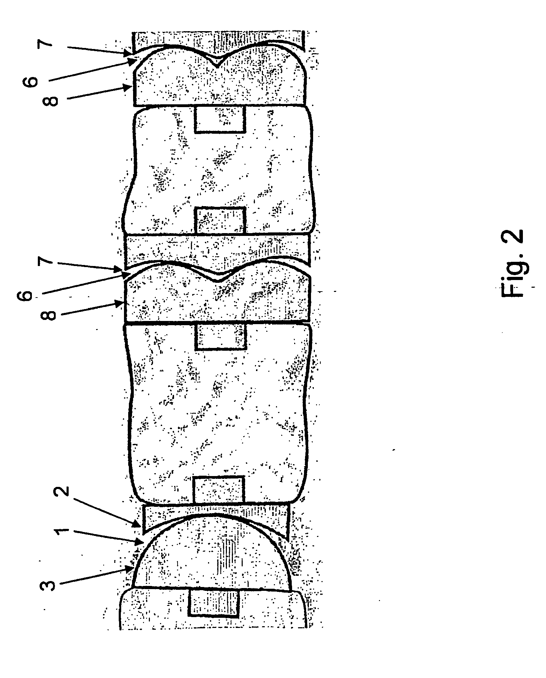

Ein erfindungsgemäßes Gelenksystem zum Einsatz eines menschlichen Fingergelenks wird anhand der Zeichnung näher dargestellt, wobei die Figur 1 einen sagittalen Schnitt und die Figur 2 einen horizontalen Schnitt des Gelenksystems zeigt. Das Gelenksystem umfasst dabei drei kinematisch gekoppelte Einzelgelenke. Jedes der in den Figuren 1 bis 4 näher dargestellten Gelenkelemente 1 und 6 ist dabei mit einer im Wesentlichen konkaven Gelenkschale 2, 7 und mit einem im Wesentlichen konvexen Gelenkkopf 3, 8 ausgestattet, die aufgrund ihrer Inkongruenz jeweils zumindest vier Freiheitsgrade aufweisen.

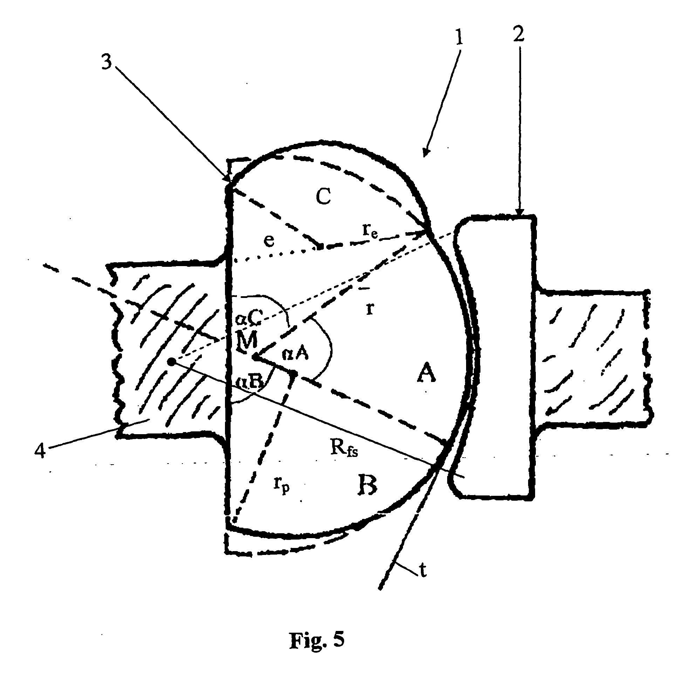

Figur 5 zeigt in einer vergrößerten Darstellung das für den Ersatz eines MCP bestimmtes künstliches Gelenkelement 1 des in Figur 1 gezeigten Gelenksystems mit seiner konkaven Gelenkschale 2 und seinem konvexen Gelenkkopf 3. Die konkave Gelenkschale 2 hat eine auf ihrer Oberfläche verlaufende gekrümmte Kontaktlinie mit einem ersten Radius R_{fs} = 10 mm und der konvexe Gelenkkopf 3 eine auf seiner Oberfläche verlaufende, ebenfalls gekrümmte Kontaktlinie mit einem abweichenden zweiten Radius r = 7mm ± 1mm, der damit wesentlich geringer als der Radius R_{fs} der Kontaktlinie der Gelenkschale 2 ist. Dem Gelenkkopf 3 liegt ein Kugelabschnitt zugrunde, der durch Gelenkkopfteil A mit dem Radius r und einem gestrichelt dargestellten Vorsprung 4 gebildet ist. Gelenkkopfteil A weist eine Schnittfläche e auf, der sich ein weiterer Kugelabschnitt als Gelenkkopfteil C mit dem Radius rₑ = 4 mm ± 1 mm anschließt. Der Schnittfläche e gegenüberliegend ist ein Gelenkkopfteil B angeformt, mit einer im Übergangsbereich zwischen dem Gelenkkopfteil A und dem Gelenkkopfteil B übereinstimmenden Tangente t der jeweiligen Oberflächen der Gelenkkopfteile A und B. Dabei ist die Mittellinie zu einem Kreis mit dem Radius rₚ = 6 mm ± 1 mm ausgebildet. Der Betrag der Winkel ergibt sich für Gelenkkopfteil A zu Winkel α_{A} = 63°± 5°, für Gelenkkopfteil B Winkel α_{B} = 65°± 5° und für Gelenkkopfteil C Winkel α_{C} = 52°± 5°. Eine vereinfachte, gestrichelte dargestellte Ausführung mit übereinstimmenden Radien r und rₚ des Gelenkkopfes 3 sind ebenfalls darstellbar. Die konkave Gelenkschale 2 ist als eine annähernd kugelförmige Schale mit einer ovalen Funktionsfläche ausgeführt. Für den Radius R_{fs} der Krümmung ergibt sich dadurch ein Betrag von ca. 10 mm und für den Winkel α_{fs} der Funktionsfläche der Betrag von 50°. Die genannten Beträge sind dabei als Richtwerte zu verstehen und können um ca. 20% variieren.

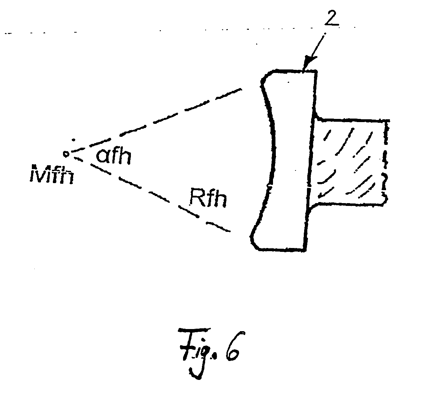

Ergänzend zeigt Figur 6 einen horizontalen Schnitt der in Figur 5 gezeigten konkaven Gelenkschale 2, mit dem Radius R_{fh} = 9 mm und dem Winkel α_{fh} = 53° wobei eine Abwandlung mit den Beträgen für Radius R_{fh} = 10 mm und dem Winkel α_{fh} = 50° ebenfalls realisierbar sind.

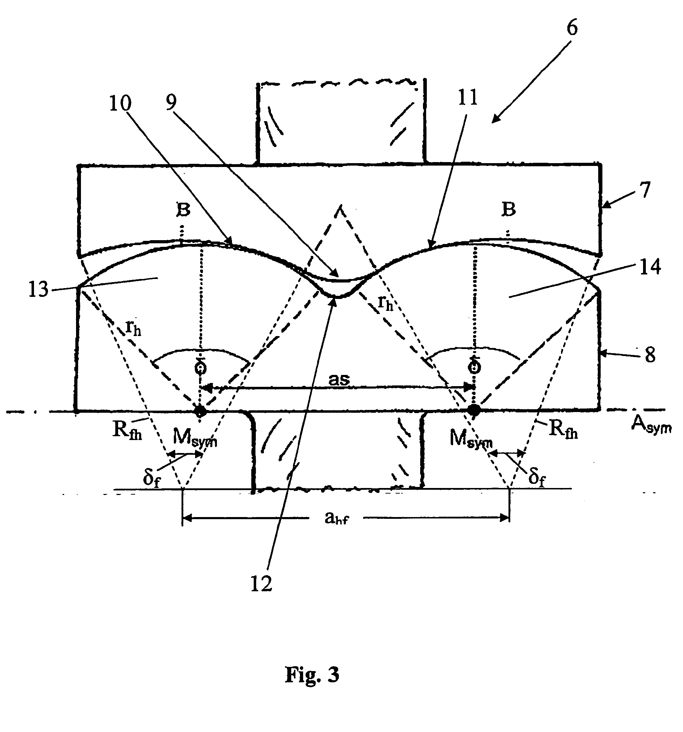

Figur 3 zeigt in einer vergrößerten Darstellung einen horizontalen Schnitt des für den Ersatz eines PIP oder DIP bestimmten künstlichen Gelenkelementes 6 des in Figur 2 gezeigten Gelenksystems mit seiner konkaven Gelenkschale 7 und seinem konvexen Gelenkkopf 8. Die Gelenkschale 7 ist dabei mit zwei konkaven, durch eine Konvexität 9 getrennten Funktionsflächen 10, 11 ausgestattet, denen jeweils ein Körper mit Rotationssymmetrie, beispielsweise ein Tonnenkörper oder eine Kugel zugrunde liegt. Die Abmessungen für den Radius R_{hf} der Funktionsflächen 10, 11 der Gelenkschale 7 betragen 6,1 mm und weisen einen Abstand a_{hf} von 7,3 mm auf. Der Winkel δ_{f} der jeweiligen Funktionsflächen 10, 11 beträgt dabei übereinstimmend 50°. Den beiden durch eine Konkavität 12 getrennten Kondylen 13, 14 des Gelenkkopfes 8 liegt ebenfalls ein Körper mit Rotationssymmetrie zur Symmetrieachse A_{sym} zugrunde. Die Abmessungen der Radien rₕ = rₛ beträgt 4,1 mm, wobei die Radienzentren einen Abstand aₛ von 7,3 mm aufweisen. Dabei ist den genannten Beträgen der Radien, Winkel und Abstände zur Ermittlung der für das DIP relevanten Beträge ein Faktor von ca. 0,7 voranzustellen.

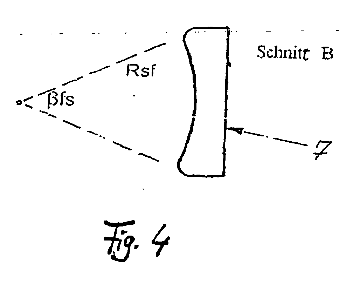

Ergänzend zeigt auch noch Figur 4 einen entlang der Linie B-B in Figur 5 geschnittene Ansicht der konkaven Gelenkschale 7 mit dem Radius R_{sf} = 5,4 mm und einem Winkel β_{fs} der Funktionsfläche von 65°.

## Patentansprüche

1. Künstliches Gelenkelement (6) zum Einsatz eines oder mehrerer Einzelgelenke in einem aus einer Kombination von mehreren kinematisch gekoppelten Einzelgelenken bestehenden Gelenksystem, insbesondere zum Einsatz bei einem menschlichen Fingergelenk, wobei jedes Gelenkelement (6) mit einer im Wesentlichen konkaven Gelenkschale (7) und mit einem im Wesentlichen konvexen Gelenkkopf (8) ausgestattet ist, wobei das Gelenkelement (6) zumindest vier Freiheitsgrade aufweist und zum Ersatz des Proximalen Interphalangeal-Gelenkes (PIP) und/oder Distalen Interphalangeal-Gelenkes (DIP) einen Gelenkkopf (8) mit zwei durch einen konkaven Bereich (12) verbundenen konvexen Bereichen (13, 14) aufweist, **dadurch gekennzeichnet, dass** der Gelenkkopf (8) sowie die Gelenkschale (7) jeweils zwei Kontaktflächen aufweisen, die in dem konvexen Bereich (13, 14) zwischen dem konkaven Bereich (12) und der maximalen distalen Erstreckung liegen.

2. Künstliches Gelenkelement (6) nach Anspruch 1, **dadurch gekennzeichnet, dass** die konkave Gelenkschale (7) eine auf ihrer Oberfläche verlaufende erste gekrümmte Kontaktlinie mit einem ersten Radius (R) aufweist und dass der konvexe Gelenkkopf (8) eine auf seiner Oberfläche verlaufende zweite gekrümmte Kontaktlinie mit einem zweiten Radius (r) aufweist, wobei der erste Radius (R) der ersten gekrümmten Kontaktlinie zumindest in einem Teilbereich zwischen den Extremlagen der Gelenkstellung größer als der zweite Radius (r) der zweiten gekrümmten Kontaktlinie ist.

3. Künstliches Gelenkelement (6) nach Anspruch 2, **dadurch gekennzeichnet, dass** die Differenz zwischen dem ersten Radius (R) und dem zweiten Radius (r) zumindest 1 mm, insbesondere zwischen 2 mm und 4 mm beträgt.

4. Künstliches Gelenkelement nach zumindest einem der Ansprüche 2 und 3, **dadurch gekennzeichnet, dass** die Differenz zwischen dem ersten Radius (R) und dem zweiten Radius (r) in der gebeugten Gelenkstellung größer als in der gestreckten Gelenkstellung ist.

5. Künstliches Gelenkelement (6) nach zumindest einem der vorhergehenden Ansprüche, **dadurch gekennzeichnet, dass** die Flächennormalen der Kontaktflächen bei jedem Flexionswinkel einen gemeinsamen Schnittpunkt aufweisen.

6. Künstliches Gelenkelement (6) nach zumindest einem der vorhergehenden Ansprüche, **dadurch gekennzeichnet, dass** das Gelenkelement (6) als ein dünnes Formteil mit einer Materialstärke zwischen 1 mm und 5 mm ausgeführt ist.

7. Ein mit einem oder mehreren künstlichen Gelenkelementen nach zumindest einem der vorhergehenden Ansprüche ausgestattetes Greifwerkzeug, welches mit einem durch eine Antriebseinheit verbundenen Zugmittel betätigbar ist, mit einer im Wesentlichen konkaven Gelenkschale und einem im Wesentlichen konvexen Gelenkkopf, wobei die konkave Gelenkschale eine gekrümmte Kontaktlinie mit einem ersten Radius aufweist, der zumindest in einem Teilbereich größer ist als ein zweiter Radius einer zweiten gekrümmten Kontaktlinie des konvexen Gelenkkopfes, wobei das Gelenkelement zumindest vier Freiheitsgrade und einen Gelenkkopf mit zwei durch einen konkaven Bereich verbundenen konvexen Bereichen aufweist, wobei der Gelenkkopf sowie die Gelenkschale jeweils zwei Kontaktflächen aufweisen, die in dem konvexen Bereich zwischen dem konkaven Bereich und der maximalen distalen Erstreckung liegen.

## Claims

1. Artificial joint element (6) for use of one or more individual joints in a joint system composed of a combination of several kinematically coupled individual joints, particularly for use in a human finger joint, each joint element (6) being equipped with a substantially concave joint shell (7) and with a substantially convex joint head (8), the joint element (6) having at least four degrees of freedom and comprising, for replacement of the proximal interphalangeal joint (PIP) and/or distal interphalangeal joint (DIP), a joint head (8) with two convex areas (13, 14) that are connected by a concave area (12), **characterized in that** the joint head (8) and the joint shell (7) each have two contact surfaces that lie in the convex area (13, 14) between the concave area (12) and the maximum distal extent.

2. Artificial joint element (1, 6) according to Claim 1, **characterized in that** the concave joint shell (7) has a first curved contact line which runs on its surface, with a first radius (R), and **in that** the convex joint head (8) has a second curved contact line which runs on its surface, with a second radius (r), the first radius (R) of the first curved contact line being greater than the second radius (r) of the second curved contact line, at least within a partial area between the extremes of the position of the joint.

3. Artificial joint element (6) according to Claim 2, **characterized in that** the difference between the first radius (R) and the second radius (r) is at least 1 mm, in particular between 2 mm and 4 mm.

4. Artificial joint element according to at least one of Claims 2 and 3, **characterized in that** the difference between the first radius (R) and the second radius (r) is greater in the flexed position of the joint than in the extended position of the joint.

5. Artificial joint element (6) according to at least one of the preceding claims, **characterized in that** the surface normals of the contact surfaces have a common point of intersection at each angle of flexion.

6. Artificial joint element (6) according to at least one of the preceding claims, **characterized in that** the joint element (6) is configured as a thin moulded part with a material thickness of between 1 mm and 5 mm.

7. Gripping tool which is equipped with one or more artificial joint elements according to at least one of the preceding claims and can be actuated by a tensioning means connected by a drive unit, with a substantially concave joint shell and with a substantially convex joint head, the concave joint shell having a curved contact line with a first radius which, at least within a partial area, is greater than a second radius of a second curved contact line of the convex joint head, the joint element having at least four degrees of freedom and comprising a joint head with two convex areas connected by a concave area, the joint head and the joint shell each having two contact surfaces which lie in the convex area between the concave area and the maximum distal extent.

## Revendications

1. Elément d'articulation artificielle (6) destiné à être utilisé dans une ou plusieurs articulations séparées dans un système articulaire constitué d'une combinaison de plusieurs articulations séparées accouplées cinématiquement et en particulier à être utilisé sur l'articulation d'un doigt humain, chaque élément d'articulation (6) étant doté d'une coque essentiellement concave d'articulation (7) et d'une tête essentiellement convexe d'articulation (8), l'élément d'articulation (6) présentant au moins quatre degrés de liberté et présentant pour remplacer l'articulation proximale inter-phalangique (PIP) et/ou l'articulation distale inter-phalangique (DIP) une tête d'articulation (8) dotée de deux parties convexes (13, 14) reliées par une partie concave (12), **caractérisé en ce que**
la tête d'articulation (8) ainsi que la coque d'articulation (7) présentent chacune deux surfaces de contact situées dans la partie convexe (13, 14) entre la partie concave (12) et l'extension distale maximale.

2. Elément d'articulation artificielle (1, 6) selon la revendication 1, **caractérisé en ce que** la coque concave d'articulation (7) présente à sa surface une première ligne de contact incurvée dotée d'un premier rayon (R) et **en ce que** la tête convexe d'articulation (8) présente à sa surface une deuxième ligne de contact incurvée dotée d'un deuxième rayon (r), le premier rayon (R) de la première ligne de contact incurvée étant, au moins dans une partie située entre les positions extrêmes de l'articulation, plus grand que le deuxième rayon (r) de la deuxième ligne de contact incurvée.

3. Elément d'articulation artificielle (6) selon la revendication 2, **caractérisé en ce que** la différence entre le premier rayon (R) et le deuxième rayon (r) est d'au moins 1 mm et est en particulier comprise entre 2 mm et 4 mm.

4. Elément d'articulation artificielle selon au moins l'une des revendications 2 et 3, **caractérisé en ce que** la différence entre le premier rayon (R) et le deuxième rayon (r) est plus grande lorsque l'articulation est en position fléchie que lorsque l'articulation est en position étendue.

5. Elément d'articulation artificielle (6) selon au moins l'une des revendications précédentes, **caractérisé en ce que** les normales aux surfaces de contact présentent un point de concours commun quel que soit l'angle de flexion.

6. Elément d'articulation artificielle (6) selon au moins l'une des revendications précédentes, **caractérisé en ce que** l'élément d'articulation (6) est configuré sous la forme d'une mince pièce moulée dont l'épaisseur du matériau est comprise entre 1 mm et 5 mm.

7. Outil de saisie doté d'un ou plusieurs éléments d'articulation artificielle selon au moins l'une des revendications précédentes, qui peut être actionné par un moyen de traction relié à une unité d'entraînement et qui présente une coque essentiellement concave d'articulation et une tête essentiellement convexe d'articulation, la coque concave d'articulation présentant une ligne de contact incurvée dotée d'un premier rayon qui, au moins dans une partie, est plus grand qu'un deuxième rayon d'une deuxième ligne de contact incurvée de la tête convexe d'articulation, l'élément d'articulation présentant au moins quatre degrés de liberté et une tête d'articulation dotée de deux parties convexes reliées par une partie concave, la tête d'articulation et la coque d'articulation présentant chacune deux surfaces de contact qui sont situées dans la partie convexe entre la partie concave et l'extension distale maximale.
